# EUROPÄISCHE PATENTANMELDUNG

(11) **EP 0 935 980 A1**
(43) Veröffentlichungstag der Anmeldung: **18.08.1999**
(21) Anmeldenummer: 99110305.2
(22) Anmeldetag: 06.05.1996
(51) Int. Cl.: A61N 5/06

(54) **Gerät zur Therapie von Rhinitis**

(30) Priorität: 16.05.1995 DE 29508077 U; 29.05.1995 DE 29508844 U; 20.09.1995 DE 29515096 U; 08.12.1995 DE 29519481 U; 08.12.1995 DE 29519482 U; 27.12.1995 DE 29520581 U
(62) Teilanmeldung aus: 98118215.7
(71) Anmelder: Wilden, Lutz, 94051 Hauzenberg (DE)
(72) Erfinder: Wilden, Lutz, 94051 Hauzenberg (DE)
(74) Vertreter: Winter, Brandl, Fürniss, Hübner, Röss, Kaiser, Polte, Kindermann Partnerschaft

(57) **Zusammenfassung**

Die vorliegende Erfindung bezieht sich allgemein auf Therapiegeräte mit einer Laserbestrahlungsvorrichtung. Insbesondere bezieht sich die Erfindung auf ein Gerät zur Therapie von Rhinitis und Akne.

## Beschreibung

Die vorliegende Erfindung bezieht sich allgemein auf Therapiegeräte mit einer Laserbestrahlungsvorrichtung. Insbesondere bezieht sich die Erfindung auf ein Gerät zur Therapie von Rhinitis und Akne gemäß dem Oberbegriff des Anspruchs 1.

Bei den von der Erfindung weitergebildeten Mundpflegegeräten handelt es sich vorzugsweise um solche Geräte, die einen Handgriff aufweisen, der mit einem in den Mund einführbaren Mundstück versehen ist, an dessen mundseitigem Ende eine Mundpflegevorrichtung sitzt. Gemäß einer Weiterbildung der Erfindung werden von dieser vorzugsweise solche Mundpflegevorrichtungen umfaßt, die elektrisch betrieben werden. Eine derartige elektrisch betriebene Mundpflegevorrichtung ist vorzugsweise eine Munddusche oder auch eine elektrische Zahnbürste. Die Vorteile derartig betriebener Mundpflegegeräte im Hinblick auf die Reinhaltung der Zähne und/oder die Massage des Zahnfleisches sind hinlänglich bekannt und sollen daher an dieser Stelle nicht weiter diskutiert werden.

In Zahnartpraxen werden in letzter Zeit vermehrt sogenannte "Low-Level-Laserbestrahlungsvorrichtungen" eingesetzt; ihr Verwendungszweck ist insbesondere die Therapie und Prophylaxe der Parodontose sowie die Therapie und die Prophylaxe von Stomatitis aphtosa, von Herpeserkrankungen der Lippen (Herpes labiales) und der Mundschleimhaut sowie von Akne. Der Zusatz "Low-Level" ("Niedrigpegel") für derartige therapeutische Laserbestrahlungsvorrichtungen wurde deshalb geprägt, weil die Ausgangsleistung bzw. Dosierung des abgegebenen Laserstrahls so bemessen ist, daß er keinerlei thermische Wirkung an dem beaufschlagten Körperteil, also insbesondere dem Zahnfleisch, hervorruft.

Es gibt bereits zahlreiche wissenschaftliche Veröffentlichungen, in denen untersucht wird, welche biologische bzw. therapeutische Wirkung kohärentes Licht geeigneter Wellenlänge auf lebendes Gewebe hat. In zahlreichen dieser Veröffentlichungen wird eine stimulierende Wirkung auf den zellulären Stoffwechsel beschrieben. Eine wesentliche Wirkung des kohärenten Lichts scheint insbesondere darin zu liegen, daß es die mitochondriale Adenosintriphosphat-Synthese ("ATP-Synthese") stimuliert. Zellschädigende Wirkungen von kohärentem Licht - sofern dessen Intensität bzw. Energiegehalt nicht zu hoch gewählt wird - sind demgegenüber noch nicht beobachtet worden, so daß das lebende Gewebe durch eine solche therapeutische Bestrahlung offensichtlich nicht geschädigt werden kann.

Trotz der anerkannt guten therapeutischen Wirksamkeit einer derartigen Low-Level-Laserbestrahlungsvorrichtung sind ihrer weiteren Verbreitung bei der Zahnfleischbehandlung dadurch Grenzen gesetzt, daß bislang lediglich in Zahnarzpraxen entsprechende Geräte zur Verfügung stehen. Die jeweilige Therapie kann daher nur vom Zahnarzt durchgeführt werden. Da die zu erzielende therapeutische Wirksamkeit in vielen Fällen eine relativ lange Behandlungsdauer erfordert, ist der Einsatz solcher Low-Level-Laserbestrahlungsvorrichtungen für die meisten Patienten vergleichsweise beschwerlich, so daß ihrer weiteren Verbreitung auch insofern Grenzen gesetzt sind.

Es wäre daher wünschenswert, ein Mundpflegegerät zu schaffen, mit dem therapeutische Wirkungen erzielbar sind.

Ein Gesichtspunkt der vorliegenden Erfindung ist somit in einer Kombination eines herkömmlichen Mundpflegegeräts mit einer Low-Level-Laserbestrahlungsvorrichtung zu sehen, wobei diese Low-Level-Laserbestrahlungsvorrichtung so im Mundpflegegerät angeordnet ist, daß der von ihr erzeugte Laserstrahl über das Mundstück in den Mund projiziert werden kann. Das erfindungsgemäße Mundpflegegerät stellt somit ein Kombinationsgerät dar, das neben der üblichen Mundpflege auch die thereapeutische Behandlung des Zahnfleisches und dergleichen ermöglicht. Ein derartiges Kombinationsgerät kann sowohl derart gestaltet sein, daß eine erfindungsgemäße Low-Level-Laserbestrahlungsvorrichtung fest in einem herkömmlichen Mundpflegegerät untergebracht ist, als auch so, daß die Low-Level-Laserbestrahlungsvorrichtung (beispielsweise in Form eines Laserstifts) lösbar am Mundpflegegerät befestigbar ist.

Neben dem unbestreitbaren Vorteil, daß mit dem erfindungsgemäßen Kombinationsgerät langwierige Besuche beim Zahnarzt vermieden werden können, hat dies den weiteren Vorteil, daß die therapeutische Behandlung wesentlich öfter und auch gleichmäßiger erfolgen kann, so daß zu erwarten ist, daß die Wirksamkeit gegenüber den in den Zahnarztpraxen installierten Laserbestrahlungsvorrichtungen gegebenenfalls sogar verbessert werden kann. Durch die häufige Anwendung des erfindungsgemäßen Mundpflegegeräts sind gute therapeutische Wirkungen selbst dann zu erwarten, wenn aus Sicherheitsgründen für den Laserstrahl eine geringe Ausgangsleistung von beispielsweise lediglich 1 bis 5 mW gewählt wird (Laserklasse IIIA).

Die erfindungsgemäß vorgesehene Low-Level-Laserbestrahlungsvorrichtung kann beispielsweise im Handgriff des Mundpflegegeräts untergebracht werden, wobei der von ihr erzeugte Laserstrahl über eine Lichtleitervorrichtung durch das Mundstück hindurchgeleitet wird und über eine am mundseitigen Ende des Mundstücks vorgesehene Linse aus diesem austritt. Da zur Erzeugung des Laserstrahls vorzugsweise ein kompakter Diodenlaser verwendet wird, werden somit die Gesamtabmessungen des Mundpflegegeräts gegenüber einem herkömmlichen Gerät kaum nennenswert vergrößert, zumal die elektrische Energieversorgung über die ohnehin vorhandene Versorgung der im Mundpflegegerät installierten Mundpflegevorrichtung, wie beispielsweise einer Munddusche oder einer elektrischen Zahnbürste, erfolgen kann. Im Falle einer als Laser-Beistellstift ausgebildeten Laserbestrahlungsvorrichtung kann die Energieversorgung über eine gemeinsame Aufladestation beispielsweise in Form einer Akku-Standkonsole erfolgen.

Die den Laserstrahl projizierende Linse ist am mundseitigen Ende des Mundstücks vorzugsweise so angeordnet, daß der Laserstrahl beim Gebrauch des Geräts im wesentlichen auf das Zahnfleisch gerichtet ist. Da die Stellung des Mundstücks beim Gebrauch einer Munddusche oder einer elektrischen Zahnbürste weitgehend definiert ist, bereitet die Auswahl einer hierfür geeigneten Position der Linse in der Praxis keine Probleme. Um auf jeden Fall sicherzustellen, daß weite Bereiche des Zahnfleisches vom Laserstrahl beaufschlagt werden, könnte gegebenenfalls daran gedacht werden, den Laserstrahl über mehrere Linsen abzustrahlen und/oder eine Linse mit breiter Fächerung zu verwenden.

Das erfindungsgemäße Mundpflegegerät wird bestimmungsgemäß insbesondere von Laien verwendet. Eine unsachgemäße Handhabung des Mundpflegegeräts kann daher insbesondere dann zu Problemen führen, wenn die Ausgangsleistung des Laserstrahls beispielsweise über 5 mW liegt und die betreffende Person den Laserstrahl auf besonders empfindliche Körperteile wie beispielsweise das Auge richtet. Um solche Gefahren auszuschließen, kann entweder daran gedacht werden, die Ausgangsleistung von vornherein auf ungefährliche Werte zu begrenzen, oder aber einen Sensor vorzusehen, der jeweils erfaßt, ob sich das mundseitige Ende des Mundstücks bzw. die Linse im Mund befindet oder nicht; wenn der Sensor erkennt, daß dies nicht der Fall ist, wird die Low-Level-Laserbestrahlungsvorrichtung von einer entsprechenden Steuervorrichtung automatisch abgeschaltet, so daß außerhalb der Mundhöhle befindliche Körperteile keinesfalls gefährdet werden können.

Die akute oder chronische sowie die allergische Rhinitis zählt zu den am häufigsten auftretenden und gleichzeitig zu den als sehr unangenehm empfundenen Erkrankungen der Nasenschleimhäute. Wenn keine Maßnahmen zur Behandlung ergriffen werden, so ist der Schnupfen zumindest mit einem sehr starken Verbrauch von Taschentüchern verbunden, was sowohl kostenträchtig als auch unhygienisch ist. Die bislang bekannten Behandlungsmethoden in Form der Verabreichung von Medikamenten oder dergleichen haben andererseits den Nachteil, daß die damit einhergehenden Nebenwirkungen wie das übermäßige Austrocknen der Schleimhäute vom medizinischen Standpunkt sehr bedenklich sind, so daß vielfach vom Gebrauch solcher Medikamente abgeraten wird.

In den Praxen von HNO-Ärzten stehen zwar bereits Geräte zur Verfügung, die in der Lage sind, durch Inhalation, Wärmebehandlung usw. eine vergleichsweise gute Heilung von Schnupfen oder Katarrh herbeizuführen, jedoch ist hierfür stets ein Besuch beim Arzt erforderlich, was entsprechend zeitaufwendig und lästig ist.

Es wäre daher wünschenswert, ein einfach handzuhabendes und gleichwohl wirksames Gerät zur Verfügung zu haben, das in der Lage ist, akute oder chronische Rhinitis wirksam zu behandeln oder sogar völlig auszuheilen.

Der Erfindung liegt die Aufgabe zugrunde, ein Gerät zur Therapie und Prophylaxe von akuter oder chronischer Rhinitis gemäß dem Oberbegriff des Anspruchs 1 zu schaffen, das eine einfache und gleichwohl wirksame therapeutische Behandlung von Rhinitis gestattet.

Diese Aufgabe wird erfindungsgemäß mit der im Kennzeichnungsteil des Anspruchs 1 angegebenen Maßnahme gelöst.

Die vorliegende Erfindung beruht auf der Erkenntnis, daß die an sich bekannte, einleitend erläuterte zellstoffwechselstimulierende Wirkung von kohärentem Licht geeigneter Energie und geeigneter Wellenlänge gegebenenfalls auch beim Auftreten von Rhinitis eine positive bzw. regenerierende und anregende biologische Wirkung entfalten könnte. Untersuchungen des Anmelders haben tatsächlich ergeben, daß durch Anwendung von kohärentem Licht beachtliche positive Auswirkungen bei der Therapie und Prophylaxe von Rhinitis festgestellt werden können. Die genannten biologischen Wirkungen des Laserlichts ermöglichen darüber hinaus auch eine positive Beeinflußung von Aknepusteln oder anderen kleinflächigen kosmetisch störenden entzündlichen Hautveränderungen oder Narben. Dies gilt in gleicher Weise für die Stimulation von Testosteron.

Unter Zugrundelegung dieser wissenschaftlichen Erkenntnis schlägt die Erfindung somit ein Gerät zur Therapie und Prophylaxe von Rhinitis sowie von Akne vor, das eine in einem Einhandgehäuse befindliche Low-Level-Laserbestrahlungsvorrichtung aufweist, die mindestens einen Laserstrahl erzeugt, der über eine in die Nasenöffnung eines Patienten einführbare Lichtleitervorrichtung auf das Naseninnere einwirkt bzw. auf Aknepustel gerichtet werden kann. Die Erfindung schafft somit ein sehr einfach und insbesondere ohne Hilfe eines Arztes oder Therapeuten bedienbares Gerät, das in jedem Haushalt vorhanden sein kann oder ggf. sogar unterwegs mitgeführt werden kann, so daß eine vergleichsweise konstante und entsprechend wirksame Behandlung durchgeführt werden kann. Insbesondere sind zeitaufwendige und teuere Besuche beim HNO-Arzt oder anderen Ärzten entbehrlich.

Wie bereits erwähnt wurde, werden die von der Erfindung verwendeten Low-Level-Laserbestrahlungsvorrichtungen bereits in zahlreichen Arztpraxen zur Behandlung von erkranktem Gewebe eingesetzt. Low-Level-Laserbestrahlungsvorrichtungen stellen somit ein bewährtes Behandlungsinstrument dar, so daß das erfindungsgemäße Gerät auf die hiermit gewonnenen Erfahrungen zurückgreifen kann, was dazu führt, daß das erfindungsgemäße Gerät trotz äußerst geringem Aufwand mit sehr hoher Zuverlässigkeit ausgestattet werden kann; insbesondere ist es möglich, die Herstellungs- und Entwicklungskosten in vergleichsweise niedrigen Grenzen zu halten.

Der sogenannte Tinnitus stellt eine chronische komplexe Innenohrstörung dar. Es handelt sich hierbei um eine Erkrankung der Hörschnecke (Cochlea). Der sogenannte chronische vestibuläre Vertigo stellt demgegenüber eine Erkrankung des Vestibularorgans (des Labyrinths) des Ohres dar. Beide Erkrankungen sind häufige Störungen des Innenohres und werden mit dem erfindungsgemäß Gerät vorzugsweise behandelt; jedoch können mit diesem Gerät ggf. auch weitere, hier nicht näher erläuterte Erkrankungen des Innenohres behandelt werden, wie z.B. die Innenohrschwerhörigkeit.

Für die betroffene Person außert sich der Tinnitus als permanenter Pfeifton bzw. als ein ununterbrochenes Summgeräusch in bestimmten Frequenzen. Dieser permanente Pfeifton ist für die betroffene Person einerseits äußerst unangenehm und kann sogar zu psychischen Störungen führen, während andererseits das Hörvermögen in dem zugeordneten Frequenzbereich entsprechend eingeschränkt ist. Aus diesem Grund werden in letzter Zeit vermehrt Anstrengungen unternommen, den Tinnitus geeigneten Therapien zu unterziehen.

Als eine der erfolgreichsten Therapien zur Tinnitusbehandlung hat sich in letzter Zeit ebenfalls die Verwendung eines Low-Level-Lasers herauskristallisiert. Im Falle der Tinnitusbehandlung mittels eines derartigen Low-Level-Lasers wurden insbesondere bei der Bestrahlung über das Mastoid (an einer ca. 2 cm hinter der Ohrmuschel befindlichen Stelle) oder des Gehörgangs bereits beachtliche Heilungserfolge erzielt. Auch der Vertigo und die Innenohrschwerhörigkeit können mit dieser Methode behandelt werden.

Die genannten Low-Level-Laservorrichtungen sind Spezialgeräte, die derzeit nur in entsprechend eingerichteten Arztpraxen oder Kliniken vorhanden sind. Der betroffene Patient muß daher für jede Innenohrstörungs- bzw. Tinnitusbehandlung eine solche Arztpraxis bzw. Klinik aufsuchen. Da eine fühlbare Heilungswirkung bei einer Low-Level-Laserbestrahlung in der Regel erst nach vergleichsweise langen Zeiträumen eintritt, muß der Patient entsprechend häufig die Arztpraxis bzw. Klinik aufsuchen. Dies ist einerseits für den Patienten lästig und hat andererseits den Nachteil, daß entsprechend hohe Behandlungskosten auftreten.

Ein weiterer wesentlicher Gesichtspunkt der vorliegenden Erfindung liegt somit darin, ein InnenohrstörungsBehandlungsgerät zu schaffen, mit dem die Behandlungskosten deutlich herabgesetzt werden können.

Die Erfindung schlägt in diesem Zusammenhang vor, eine Low-Level-Laserbestrahlungsvorrichtung vorzusehen, die mittels einer geeigneten Befestigungsvorrichtung lösbar derart am Ohr eines Patienten befestigt werden kann, daß der Laserstrahl auf mindestens einen vorbestimmten Bereich des Ohres einwirkt. Der Kerngedanke dieser Maßnahme ist somit darin zu sehen, eine Low-Level-Laserbestrahlungsvorrichtung zu schaffen, die der Patient für die Dauer der Behandlung ständig mit sich trägt, so daß eine entsprechend intensive Behandlung erfolgt, was erwarten läßt, daß die Heilungsaussichten mit dem erfindungsgemä-ßen Gerät sogar noch höher sind, als mit den in Arztpraxen bzw. Kliniken vorhandenen herkömmlichen Low-Level-Laservorrichtungen. Ein weiterer Vorteil des erfindungsgemäßen Innenohrstörungs-Behandlungsgeräts liegt darin, daß es im Prinzip ausreicht, wenn das Gerät von einem entsprechenden Therapeuten zu Beginn der Behandlung angepaßt wird; weitere Arztbesuche sind für den Patienten dann nicht mehr notwendig, so daß die Therapie für den Patienten entsprechend bequemer ist. Die mit den Arzt- bzw. Klinikbesuchen verbundenen Behandlungskosten entfallen ebenfalls, so daß insgesamt große Kostenvorteile erzielt werden.

Die Low-Level-Laserbestrahlungsvorrichtung des erfindungsgemäßen Innenohrstörungs-Behandlungsgeräts kann in der Befestigungsvorrichtung selbst untergebracht sein, wobei in diesem Fall vorzugsweise eine Lichtleitervorrichtung vorgesehen ist, über die der von der Laserbestrahlungsvorrichtung abgegebene Laserstrahl ggf. einer Linse zugeführt wird, aus der der Laserstrahl austritt und auf den/die vorbestimmten Bereich(e) einwirkt. Der Lichtleiter besteht vorzugsweise aus einem Material, das es dem das Gerät anpassenden Therapeuten gestattet, die Position des Lichtaustritts, d.h. den vorbestimmten Wirkungsbereich, durch Verbiegen, Verschwenken oder dergleichen einzustellen. Gegebenenfalls kann zwischen der Befestigungsvorrichtung und der Lichtleitervorrichtung auch eine Verstellvorrichtung vorgesehen werden, die es gestattet, die Länge und/oder Richtung der Lichtleitervorrichtung durch Verschrauben oder dergleichen zu ändern.

Alternativ kann es sich bei der Low-Level-Laserbestrahlungsvorrichtung des erfindungsgemäßen Innenohrstörungs-Behandlungsgeräts auch um eine separate Einheit handeln, deren Laserstrahl über eine flexible Lichtleitervorrichtung ggf. einer an ihrem Ende sitzenden Linse zugeführt wird, wobei in diesem Fall lediglich die Linse und/oder das Ende der Lichtleitervorrichtung mittels der Befestigungsvorrichtung am Ohr befestigt wird. Bei dieser Variante der Erfindung wird die Laserbestrahlungsvorrichtung beispielsweise in die Hemdtasche, in die Hosentasche oder an den Gürtel des Patienten gesteckt. Diese Variante der Erfindung wird daher insbesondere dann eingesetzt, wenn die verwendete Low-Level-Laserbestrahlungsvorrichtung und/oder deren Batteriestromversorgung vergleichsweise groß und/oder schwer ist.

Ein weiterer, wesentlicher Aspekt bei diesem Gerät liegt darin, daß als Befestigungsvorrichtung eine bereits vorhandene Einrichtung verwendet wird. Bei einer solchen Einrichtung kann es sich insbesondere um ein Brillengestell, ein Hörgerät, einen Tinnitusmasker oder um ein Kombinationsgerät aus einem Tinnitusmasker und einem Hör-gerät handeln (ein Tinnitusmasker ist ein kleiner Lautsprecher, der ein Geräusch in der Frequenz des Tinnitus erzeugt, so daß der Patient den Eindruck hat, beim Tinnitus würde es sich um ein externes Geräusch handeln). Selbstverständlich ist es jedoch auch möglich, für das erfindungsgemäße Innenohrstörungs-Behandlungsgerät eine eigene Befestigungsvorrichtung vorzusehen, wie beispielsweise einen am Ohr befestigbaren Bügel, ein in das Ohr einführbares Teil (vergleichbar einem Innenohr-Hörgerät) oder einen auf den Kopf aufstülpbaren Bügel nach Art eines Kopfhörers.

Störungen des Zentralnervensystems eines Menschen treten bekanntlich in mehr oder weniger gravierender Form auf. In diesem Zusammenhang ist insbesondere die bekannte Alzheimerkrankheit zu nennen. Viele Menschen sind darüber hinaus von einer allgemeinen oder spezifischen Hirnleistungsschwäche betroffen, leiden an Depressionen, Konzentrationsstörungen usw. Es wäre daher wünschenswert, ein einfach handzuhabendes und gleichwohl wirksames Gerät zur Verfügung zu haben, das durch geeignete Stimulierung des Zentralnervensystems die vorstehend genannten Erkrankungen wirksam behandeln oder sogar völlig ausschalten kann. Ein derartiges Gerät könnte gegebenenfalls sogar zur allgemeinen zerebralen Leistungssteigerung verwendet werden.

Ein weiterer wesentlicher Gesichtspunkt der vorliegenden Erfindung liegt somit darin, ein Gerät zur Stimulierung des Zentralnervensystems zu schaffen, das eine einfache und gleichwohl wirksame therapeutische Behandlung zerebraler Erkrankungen gestattet.

Der vorliegende Aspekt der Erfindung beruht auf der Erkenntnis, daß die bekannte zellstoffwechselstimulierende Wirkung von kohärentem Licht geeigneter Energie und geeigneter Wellenlänge gegebenenfalls auch im Zentralnervensystem eine positive bzw. regenerierende und anregende biologische Wirkung entfalten könnte. Untersuchungen des Anmelders haben tatsächlich ergeben, daß durch Anwendung von kohärentem Licht beachtliche positive Auswirkungen auf das Zentralnervensystem festgestellt werden können. Es ist somit zu erwarten, daß die von der Erfindung vorgeschlagene Anwendung von kohärentem Licht zur Stimulierung des Zentralnervensystems geeignet ist, die eingangs genannten Krankheiten, wie insbesondere die Alzheimerkrankheit, die allgemeine oder spezifische Hirnleistungsschwäche, Depressionen, Konzentrationsstörungen usw., therapeutisch wirksam zu behandeln oder sogar völlig auszuschalten.

Unter Zugrundelegung dieser wissenschaftlichen Erkenntnis schlägt die Erfindung somit ein Gerät zur Stimulierung des Zentralnervensystems vor, das eine Low-Level-Laserbestrahlungsvorrichtung aufweist, die mindestens einen Laserstrahl erzeugt, der auf mindestens einen vorbestimmten Bereich der Haut des Patienten, vorzugsweise in unmittelbarer Nähe des zu stimulierenden Zentralnervensystems, einwirkt. Aufgrund der bereits zahlreichen Verwendung derartiger Low-Level-Laserbestrahlungsvorrichtungen (siehe die obigen Ausführungen) stellen diese ein bewährtes Behandlungsinstrument dar, so daß das erfindungsgemäße Gerät auf die hiermit gewonnenen Erfahrungen zurückgreifen kann, was dazu führt, daß das erfindungsgemäße Gerät trotz äußerst geringem Aufwand mit sehr hoher Zuverlässigkeit ausgestattet werden kann; auch die Herstellungs- und Entwicklungskosten halten sich dadurch in vergleichsweise niedrigen Grenzen.

Ein weiterer Gesichtspunkts liegt darin, daß das Zentralnervensystem-Stimulierungsgerät in der Lage ist, verschiedene Bereiche des Zentralnervensystems entweder gleichzeitig oder - je nach Indikationsstellung - partiell mit dem Laserstrahl bzw. den Laserstrahlen der Low-Level-Laserbestrahlungsvorrichtung von außen zu behandeln. Dies kann beispielsweise dadurch erreicht werden, daß die Low-Level-Laserbestrahlungsvorrichtung auf den Kopf des Patienten einwirkt. Zu diesem Zweck ist es von besonderem Vorteil, wenn ein helm- oder haubenartiger Aufsatz für den Kopf des Patienten vorgesehen wird, wobei dieser Aufsatz vorzugsweise eine Vielzahl von Laserstrahl-Sendeelementen trägt, deren Laserstrahlen ins Innere des Aufsatzes gerichtet sind. Hierdurch ist es möglich, das Zentralnervensystem relativ gleichmäßig von außen zu behandeln; gleichwohl kann auf sehr einfache Weise eine gezielte bereichsweise Behandlung erfolgen, indem die entsprechenden Sendeelemente selektiv aktiviert werden.

Der Aufsatz kann beispielsweise an einem schwenkbaren Halter befestigt werden, so daß er auf einfache Weise nach Art einer Trockenhaube über den Kopf des Patienten gestülpt werden kann, wobei auch der Abstand der Sendeelemente zur Kopfhaut des Patienten äußerst einfach eingestellt werden kann. Zur Einstellung eines definierten Abstands zur Kopfhaut können alternativ oder zusätzlich zu dieser Maßnahme im Inneren des Aufsatzes Abstandshalter vorgesehen werden, die die Innenwand des Aufsatzes und damit die Laserstrahl-Sendeelemente in einem vorbestimmten Abstand zur Kopfhaut des Patienten halten. Derartige Abstandshalter sind insbesondere dann nutzvoll, wenn der erfindungsgemäße Aufsatz ohne Halter aufgestülpt wird, d.h. eine Art Helm darstellt.

Die Laserstrahl-Sendeelemente können beispielsweise jeweils aus einer Laserdiode gebildet werden, die entweder jeweils von einer eigenen Batterie oder aber von einer zentralen Stromversorgungseinrichtung gespeist werden. Alternativ hierzu kann jedes Laserstrahl-Sendeelement aus einer Lichtleitervorrichtung bestehen, an deren Ende gegebenenfalls eine Linse sitzt. Am anderen Ende werden alle Laserstrahl-Sendeelemente beispielsweise aus einer gemeinsamen Laserlichtquelle gespeist. Alternativ hierzu kann für jedes oder zumindest für einen Teil der Laserstrahl-Sendeelemente eine eigene Laserlichtquelle vorgesehen werden, so daß die Möglichkeit besteht, einige oder alle Laserstrahl-Sendeelementen mit anderer Frequenz und/oder Leistung zu betreiben. Die Laserlichtquellen können entweder Teil der Haube sein oder in einem externen Gerät angeordnet werden.

Obgleich der genannte helm- oder haubenartige Aufsatz die bevorzugte Art der therapeutischen Behandlung des Kopfs eines Patienten darstellt, kann die Low-Level-Laserbestrahlungsvorrichtung auch auf jede andere Art auf die zu beaufschlagenden Bereiche des Zentralnervensystems einwirken. So ist es zum Beispiel möglich, die Low-Level-Laserbestrahlungsvorrichtung an Schwenkarmen, Fühlern, externen Strahlern oder dergleichen anzubringen. Gegebenenfalls ist es sogar möglich, die Low-Level-Laserbestrahlungsvorrichtung als Teil eines Ganzkörperbehandlungsgeräts nach Art einer Sonnenbank auszubilden. In jedem Fall sollten jedoch Maßnahmen ergriffen werden, die sicher verhindern, daß die Intensität der abgegebenen Laserstrahlen nur so hoch ist, daß empfindliche Körperteile wie insbesondere das Auge hierdurch nicht geschädigt werden.

Das von der Erfindung geschaffene Gerät zur Stimulierung des Zentralnervensystems kann sowohl als therapeutisches (medizinisch-technisches) Gerät als auch als solches Gerät konzipiert sein, das vom Laien in Eigenverantwortung benutzt wird. Ferner ist es möglich, ein solches Gerät als Münzgerät auszubilden, das bei Einwurf eines geeigneten Münzbetrages eine vorbestimmte Zeit arbeitet.

Es ist allgemein bekannt, daß bei längerer Bettlägrigkeit (wie beispielsweise im Altenheim, nach längerem Krankenhausaufenthalt oder bei ambulanter Pflege) das Problem des Wundliegens auftritt; dieses in Fachkreisen als Dekubitus bezeichnete Phänomen stellt eine ernsthafte Erkrankung der betroffenen Körperbereiche dar und bedarf daher einer sorgfältigen Behandlung. Gleichwohl hat es sich bislang als schwierig erwiesen, den Dekubitus geeignet zu behandeln; auch eine Vorbeugung oder Prophylaxe war bisher kaum möglich

Es wäre daher wünschenswert, ein einfach handzuhabendes und gleichwohl wirksames Gerät zur Verfügung zu haben, das durch geeignete Stimulierung dem Auftreten von Dekubitus vorbeugen oder diesen nach seinem Auftreten wirksam behandeln oder sogar völlig ausschalten kann.

Ein weiterer wesentlicher Gesichtspunkt der vorliegenden Erfindung liegt somit darin, ein Gerät zur Therapie und Prophylaxe von Dekubitus zu schaffen, das eine einfache und gleichwohl wirksame therapeutische Behandlung von Dekubitus gestattet.

Die vorliegende Erfindung macht sich erneut die Erkenntnis zunutze, daß die an sich bekannte zellstoffwechselstimulierende Wirkung von kohärentem Licht geeigneter Energie und geeigneter Wellenlänge gegebenenfalls auch beim Auftreten von Dekubitus eine positive bzw. regenerierende und anregende biologische Wirkung entfalten könnte. Untersuchungen des Anmelders haben tatsächlich ergeben, daß durch Anwendung von kohärentem Licht beachtliche positive Auswirkungen bei der Therapie und Prophylaxe von Dekubitus festgestellt werden können.

Unter Zugrundelegung dieser wissenschaftlichen Erkenntnis schlägt die Erfindung somit ein Gerät zur Therapie und Prophylaxe von Dekubitus vor, das eine Low-Level-Laserbestrahlungsvorrichtung aufweist, die mindestens einen Laserstrahl erzeugt, der auf bestimmte Bereich der Haut eines Patienten einwirkt.

Besonders vorteilhaft läßt sich das erfindungsgemäße Gerät dann einsetzen, wenn eine Vielzahl von Laserstrahl-Sendeelementen vorgesehen werden, die sich entsprechend auf verschiedene Bereiche der Haut des Patienten richten lassen, so daß eine gleichmäßige Behandlung der gesamten betroffenen Hautoberfläche möglich ist. Weiterhin ist es von Vorteil, eine Zeitschaltuhr vorzusehen, die nach Ab-lauf einer vorwählbaren Zeitspanne die Laserstrahl-Sendeelemente deaktiviert. Auf diese Weise kann jeder Patient einer vorbestimmten Behandlungsdauer von beispielsweise 30 Minuten unterzogen werden, ohne daß Pflegepersonal anwesend sein muß.

Um zu erreichen, daß die der Dekubitus-Therapie zu unterziehenden Hautbereiche gleichmäßig mit den Laserstrahlen beaufschlagt werden, ist es möglich, eine Streulinse zur Fächerung des Laserstrahls zu verwenden. Alternativ hierzu oder zusätzlich kann daran gedacht werden, den Abstrahlwinkel der Laserstrahl-Sendeelemente motorisch in der Weise zyklisch zu verändern, daß der wunde Bereich der Haut des Patienten in zyklischer Folge mit den Laserstrahlen beaufschlagt wird.

Dieses Gerät kann insbesondere auch zur Heimbehandlung (also zur Selbstbehandlung durch den Patienten) von chronischen Hauterkrankungen (Hauttherapie) sowie zur Thymusstimulation verwendet werden.

Sowohl bei in Wohnungen verwendeten Zierpflanzen als auch beim gewerblichen Züchten von Pflanzen ist es oberstes Ziel, das Wachstum der betreffenden Pflanzen so weit wie möglich zu fördern. Weiterhin soll die Widerstands-kraft der Pflanzen gegenüber Erkrankungen oder Schädlingen erhöht werden. In aller Regel werden diese Ziele chemische Düngemittel und/oder durch chemische Pflanzenschutzmittel erreicht. Die Nachteile der Verabreichung solcher Mittel sind hinlänglich bekannt und bedürfen somit keiner weiteren Erläuterung. Um den Einsatz derartiger chemischer Mittel zu verhindern oder doch zumindest zu reduzieren wurde daher bereits überlegt, das Pflanzenwachstum auf alternative Weise zu fördern, so beispielsweise durch biologische Stimulierung. Bislang sind gleichwohl noch keine Geräte bekannt geworden, die eine zuverlässige biologische Stimulierung von Pflanzen ermöglichen.

Ein weiterer wesentlicher Gesichtspunkt der vorliegenden Erfindung liegt somit darin, ein Gerät zur Biostimulierung von Pflanzen zu schaffen, das eine einfache und gleichwohl wirksame Biostimulierung von Pflanzen gestattet.

Erfindungsgemäß wurde erstmals erkannt, daß die zellstoffwechselstimulierende Wirkung von kohärentem Licht geeigneter Energie und geeigneter Wellenlänge gegebenenfalls auch im Zellensystem von Pflanzen eine positive bzw. regenerierende und anregende biologische Wirkung entfalten kann. Untersuchungen des Anmelders haben tatsächlich ergeben, daß durch Anwendung von kohärentem Licht beachtliche positive Auswirkungen auf das Zellensystem von Pflanzen festgestellt werden können. Es ist somit zu erwarten, daß die von der Erfindung vorgeschlagene Anwendung von kohärentem Licht zur Biostimulierung von Pflanzen geeignet ist, das Wachstum der betreffenden Pflanzen stark zu fördern und auch die Widerstandskraft der Pflanzen gegenüber Erkrankungen oder Schädlingen zu erhöhen.

Unter Zugrundelegung dieser wissenschaftlichen Erkenntnis schlägt die Erfindung somit ein Gerät zur Biostimulierung von Pflanzen vor, das eine Low-Level-Laserbestrahlungsvorrichtung aufweist, die mindestens einen Laserstrahl erzeugt, der auf bestimmte Bereiche der Oberfläche der Pflanzen einwirkt. Das erfindungsgemäße Gerät kann wirksam sowohl für Zierpflanzen oder dergleichen als auch für gewerbliche Zwecke wie beispielsweise in Gewächshäusern eingesetzt werden. Jedenfalls können chemische Mittel in erheblichen Mengen eingespart werden, wobei gleichwohl eine gute Wachstumsstimulierung und hohe Widerstandskraft gegen Erkrankungen erzielbar ist.

Besonders vorteilhaft läßt sich das erfindungsgemäße Gerät dann einsetzen, wenn eine Vielzahl von Laserstrahl-Sendeelementen vorgesehen werden, die sich entsprechend auf verschiedene Bereiche der zu stimulierenden Pflanze(n) richten lassen, so daß eine gleichmäßige Behandlung der gesamten Oberfläche der Pflanze(n) möglich ist. Weiterhin ist es von Vorteil, eine manuell bedienbare oder automatisch arbeitende Steuereinrichtung vorzusehen, die in Abhängigkeit von der Wachstumsphase der betreffenden Pflanze(n) eines oder mehrere Laserstrahl-Sendeelemente mit jeweils geeigneter Wellenlänge aktiviert und/oder deren Ausgangsleistung ändert. Somit ist eine optimale Anpassung des erfindungsgemäßen Geräts an das Wachstum der Pflanze(n) möglich.

Untersuchungen haben gezeigt, daß mit den erfindungsgemäßen Geräten insbesondere dann große Erfolge erzielbar sind, wenn die Low-Level-Laserbestrahlungsvorrichtung einen Laserstrahl mit einer Wellenlänge erzeugt, die sich vom Ultraviolettbereich bis hin zum nahen Infrarotbereich erstreckt, also von circa 180 nm bis etwa 1000 nm, wobei die Ausgangsleistung bei den zur Eigenbehandlung vorgesehenen Geräten vorzugsweise zwischen 1 mW und 5 mW liegen sollte; das Gerät entspricht somit vorzugsweise der Laserspezifikation Klasse IIIA. Wenn die Geräte demgegenüber für den professionellen Gebrauch durch geschulte Therapeuten bestimmt sind, werden Laserbestrahlungsvorrichtungen mit Ausgangsleistungen bis maximal 500 mW eingesetzt.

Gegebenenfalls kann daran gedacht werden, das Gerät mit einer Hand-Einstellvorrichtung zu versehen, mittels der die Ausgangsleistung der Low-Level-Laserbestrahlungsvorrichtung und/oder die Wellenlänge des Laserstrahls vom Patienten auf einen wählbaren Wert eingestellt werden kann. Die Low-Level-Laserbestrahlungsvorrichtung kann entweder im kontinuierlichen Betrieb oder auch im pulsierenden Betrieb arbeiten, wobei die gewünschte Arbeitsweise gegebenenfalls mit einem entsprechenden Wählschalter eingestellt werden kann.

Die Erfindung wird nachstehend anhand der Beschreibung von Ausführungsbeispielen unter Bezugnahme auf die Zeichnung näher erläutert. Es zeigen:
Fig.1 eine Ausführungsform des Mundpflegeräts;
Fig.2 eine Ausführungsform des Gerät (200) zur Therapie von Rhinitis und Akne, das auch zur Stimulation von Testosteron im Hoden eingesetzt werden kann;
Fig.3A und 3B zwei Ausführungsformen eines Innenohrstörungs-Behandlungsgeräts zur Therapie einer chronischen komplexen Innenohrstörung;
Fig.4A und 4B zwei Ausführungsformen eines Geräts zur Stimulierung des Zentralnervensystems eines Patienten;
Fig.5 eine Ausführungsform eines Geräts zur Therapie und Prophylaxe von Dekubitus; und
Fig.6A und 6B zwei Ausführungsformen eines Geräts zur Biostimulation von Pflanzen.

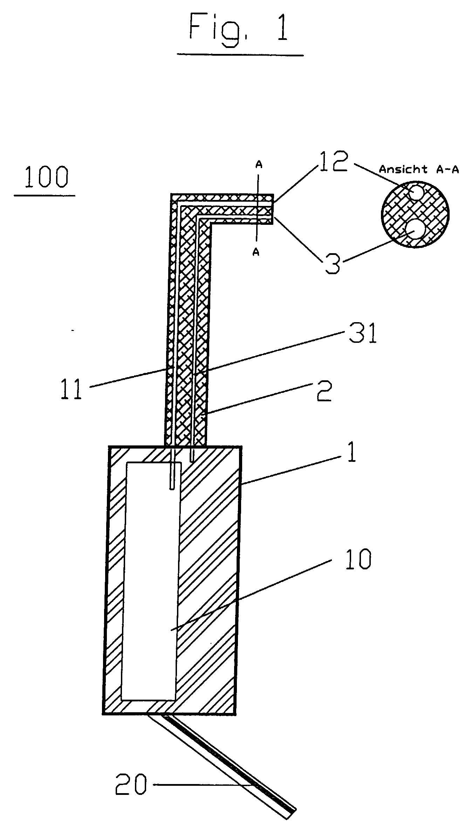

In Fig.1 ist schematisch ein Mundpflegegerät in Form einer Munddusche 100 gezeigt. Diese Munddusche 100 weist einen Handgriff 1 auf, an dessen einem Ende ein rohrförmiges Mundstück 2 sitzt, während an seinem anderen Ende eine vorzugsweise flexible Versorgungsleitung 20 vorgesehen ist, die sowohl zur Wasserzufuhr als auch zur Stromversorgung dient. Die (nicht gezeigte) Pumpe der Munddusche kann sowohl in einem (nicht gezeigten) Wasserbehälter als auch im Handgriff 1 angeordnet sein. Der Ein/Aus-Schalter der Munddusche 100 kann ebenfalls entweder am Wasserbehälter oder am Handgriff 1 vorgesehen sein, wobei die letztere Lösung eine einfachere Handhabung des erfindungsgemäßen Mundpflegegeräts ermöglicht.

Wie aus der Fig.1 zu erkennen ist, verläuft im rohrförmigen Mundstück 2 ein Wasserkanal 31, der einer lediglich schematisch gezeigten Düse 3 das von der Pumpe unter Druck gesetzte Wasser zuführt. Durch das von der Düse 3, gegebenfalls pulsierend, abgegebene Wasser werden die damit beaufschlagten Zähne gereinigt sowie das Zahnfleisch massiert. Die Arbeitsweise einer derartigen Munddusche ist im übrigen bekannt, so daß nähere Erläuterungen entbehrlich erscheinen.

Erfindungsgemäß ist innerhalb des Handgriffs 1 eine Low-Level-Laserbestrahlungsvorrichtung 10 angeordnet, die einen Laserstrahl erzeugt, der über einen Lichtleiter 11, der innerhalb des rohrförmigen Mundstücks 2 im wesentlichen parallel zum Wasserkanal 31 verläuft, zu einer Linse 12 geleitet wird, über die er schließlich austritt. Wie insbesondere aus der Schnittansicht A-A zu erkennen ist, ist die Linse 12 etwas oberhalb der Düse 3 derart angeordnet, daß der Laserstrahl beim Gebrauch der Munddusche im wesentlichen auf das Zahnfleisch gerichtet ist. Gegebenenfalls ist es möglich, eine Linse mit einem großen Streubereich zu verwenden, so daß entsprechend große Bereiche des Zahnfleisches vom Laserstrahl erfaßt werden. Weiterhin ist es möglich, den Laserstrahl über mehrere Linsen austreten zu lassen.

Die Low-Level-Laserbestrahlungsvorrichtung 10 wird über einen (nicht gezeigten) Schalter aktiviert.

Anstelle der im Ausführungsbeispiel gezeigten Munddusche kann es sich bei der erfindungsgemäßen Mundpflegevorrichtung auch um eine elektrische Zahnbürste handeln. Die Low-Level-Laserbestrahlungsvorrichtung 10 und insbesondere ihre Linse 12 werden hierbei in analoger Weise angeordnet. Die Low-Level-Laserbestrahlungsvorrichtung 10 kann aber auch ein separates bzw. eigenständiges Teil beispielsweise in Form eines Laserstifts sein, der dem betreffenden Mundpflegesystem beigestellt wird.

Obgleich dies nicht gezeigt ist, kann für die Low-Level-Laserbestrahlungsvorrichtung 10 ein mechanischer oder optoelektronischer Sensor vorgesehen werden, der erfaßt, ob sich das mundseitige Ende des Mundstücks 2 bzw. die Linse 12 im Mund befindet oder nicht. Wenn dieser Sensor erkennt, daß dies nicht der Fall ist, das Mundstück 2 sich also außerhalb der Mundhöhle befindet, wird von einer (nicht gezeigten) Steuervorrichtung die Low-Level-Laserbestrahlungsvorrichtung abgeschaltet, so daß kein Laserstrahl erzeugt wird, der gegebenenfalls Schaden anrichten könnte.

Die erfindungsgemäße Low-Level-Laserbestrahlungsvorrichtung verwendet vorzugsweise einen Diodenlaser. Jedoch ist es möglich, andere geeignete Lasererzeugungsvorrichtungen zu verwenden. Die Wellenlänge des erzeugten Laserlichts liegt im Bereich zwischen 630 nm und 830 nm, also im Bereich des rötlichen Lichts. Wenn der Bereich der Wellenlänge bis auf 450 nm ausgedehnt wird, wird auch grünliches Licht abgegeben.

Gegebenenfalls ist es auch möglich, die Low-Level-Laserbestrahlungsvorrichtung 10 nicht nur kontinuierlich, sondern auch pulsierend oder in anderer Weise mit wechselnder Ausgangsleistung zu betreiben, falls hierdurch die therapeutische Wirksamkeit gesteigert werden kann. Eine entsprechende Regelelektronik kann ohne weiteres im Handgriff 1 integriert werden.

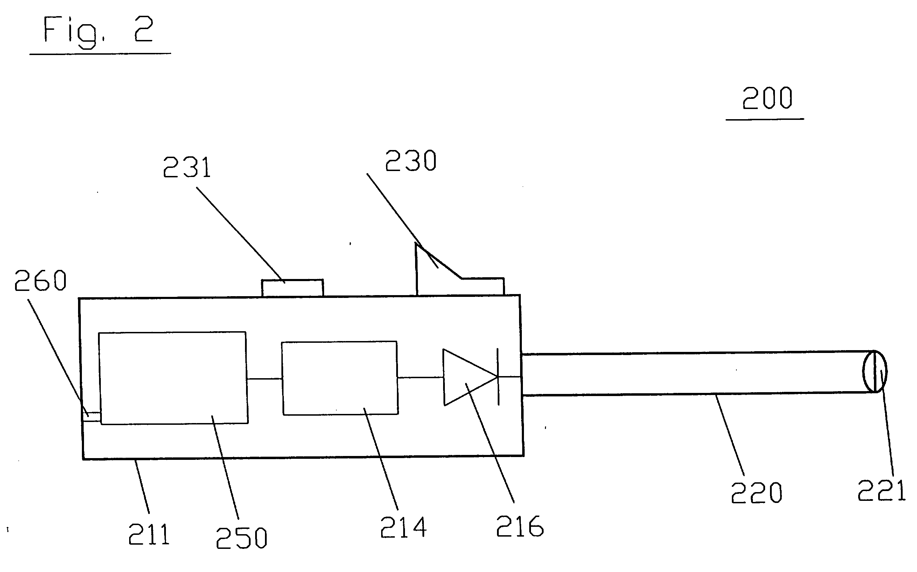

In Fig.2 ist eine Ausführungsform des erfindungsgemäßen Geräts 200 zur Therapie und Prophylaxe von Rhinitis und Akne gezeigt.

Wie aus der Fig.2 hervorgeht, weist das erfindungsgemäße Gerät 200 ein Gehäuse 211 auf, das beispielsweise aus Metall oder einem geeigneten Kunststoffmaterial besteht. Das Gehäuse 211 ist nach Art eines Handgriffs etwa rund gefertigt, so daß das Gerät 200 in einer Hand gehalten und bedient werden kann.

Im Inneren des Gehäuses 211 ist eine wiederaufladbare Batterie 250 zur Stromversorgung vorgesehen, die über ei-ne lediglich schematisch gezeigte Ladevorrichtung 260 am Stromnetz geladen werden kann. Alternativ kann es sich bei der Batterie auch um eine herkömmliche, nicht-wiederaufladbare Batterie handeln; weiterhin ist es möglich, das Gerät über eine Leitung (und ggf. einen zusätzlichen Trafo) direkt am Stromnetz zu betreiben.

Im Inneren des Gehäuses 211 ist eine Low-Level-Laserbestrahlungsvorrichtung vorgesehen, die aus einer Ansteuerelektronik 214, die von der Batterie 250 gespeist wird, und einem Laserstrahl-Sendeelement in Form einer Laserdiode 216 besteht. Das von der Laserdiode 216 erzeugte Laserlicht wird in eine längliche, z.B. in die Nasenöffnung eines Patienten einführbare Lichtleitervorrichtung 220 eingespeist. Am Ende der Lichtleitervorrichtung 220 sitzt eine Streulinse 221 zur Fächerung des von der Laserdiode 216 abgegebenen Laserstrahls. Die Ausgangsleistung der Laserdiode 216 liegt zwischen 1 mW und 5 mW und entspricht damit der Klasse IIIA. Die Laserdiode 16 erzeugt einen Laserstrahl mit einer möglichen Wellenlänge im Bereich von 180 nm bis 1000 nm.

Zum Einschalten des Geräts ist ein Ein/Aus-Schalter 230 vorgesehen, während die Ausgangsleistung des Laserstrahl-Sendeelements 216 mittels eines Drehreglers 231 eingestellt werden kann.

Wie aus der Fig.2 ersichtlich ist, kann das Gerät 200 mit einer Hand so ergriffen werden, daß das Ende der Lichtleitervorrichtung 220 auf einfachste Weise in die Nase eingeführt werden kann. Das Naseninnere kann somit wirksam mit dem Laserlicht beaufschlagt werden. Somit kann eine gleichmäßige und ausreichende therapeutische Behandlung der erkrankten Nasenschleimhäute mit wenigen Handgriffen erreicht werden.

Das vorstehend beschriebene Gerät 200 kann erfindungsgemäß auch zur Behandlung von Akne verwendet werden. Ferner ist es möglich, dieses Gerät zur Stimulation von Testosteron im Hoden einzusetzen. Aufgrund der hierdurch hervorgerufenen Erhöhung der Testosteron-Produktion wird eine Verbesserung der erektielen Potenz des betreffenden Patienten erreicht.

Um dem Patienten eine möglichst einfache bzw. komfortable Handhabung des erfindungsgemäßen Testosteron-Stimulationsgeräts zu ermöglichen, ist es ferner möglich, dieses als flexibles tragbares Netz oder als Kissen auszubilden, in dem die Low-Level-Laserbestrahlungsvorrichtung angeordnet ist und den Hoden über mehrere gleichmäßig verteilte Laserstrahl-Sendeelemente beaufschlagt. Ein solches Kissen kann um den Hoden herum angelegt und dann z.B. im Sitzen beispielsweise beim Lesen oder Fernsehen getragen werden.

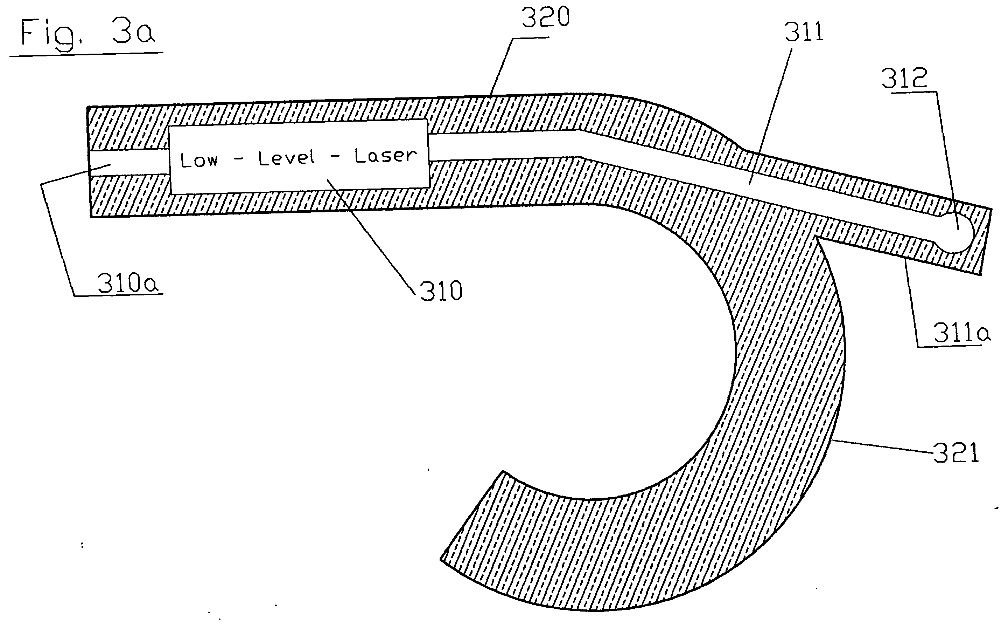

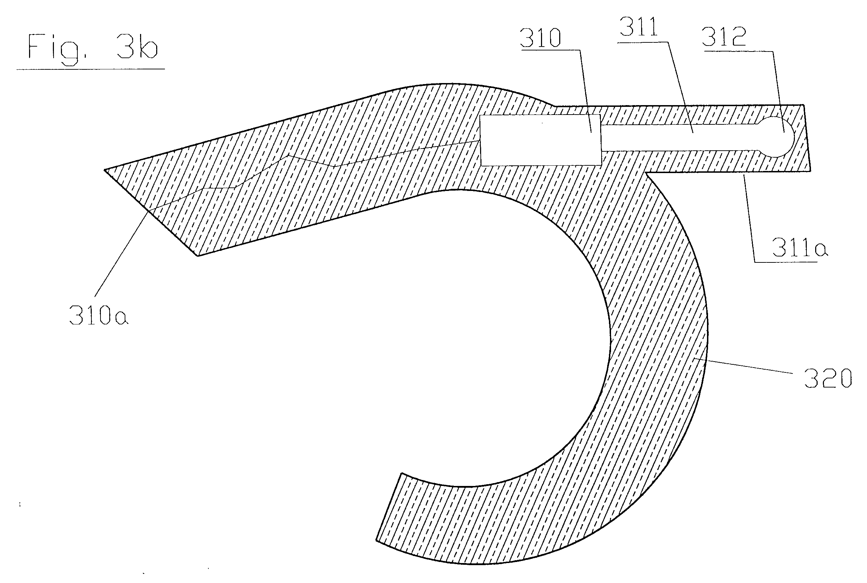

Gemäß Fig.3A weist eine erste Ausführungsform des erfindungsgemäßen Innenohrstörungs-Behandlungsgeräts eine Low-Level-Laserbestrahlungsvorrichtung 310 auf, die in das Innere eines Brillengestells 320 in der Nähe des Bügels 321 desselben eingebaut ist. Die Laserbestrahlungsvorrichtung 310 wird über Kabel 310a aus einer (nicht gezeigten) Stromquelle in Form einer Batterie oder dergleichen gespeist. Der von der Laserbestrahlungsvorrichtung 310 abgegebene Laserstrahl wird über eine Lichtleitervorrichtung 311 einer Linse 312 zugeführt, aus der er aus-tritt und auf den darunter liegenden Bereich einwirkt. Die Lichtleitervorrichtung 311 verläuft austrittsseitig in einem etwa rohrförmigen Ansatz 311a, der aus einem elastisch verformbaren Material besteht, so daß die Position der Linse 312 und damit der Wirkbereich des erfindungsgemäßen Innenohrstörungs-Behandlungsgeräts geändert bzw. an den Patienten angepaßt werden kann. Da diese erste Ausführungsform in eine Brille integriert ist, wird somit ein Kombinationsgerät geschaffen, das bei einem Brillenträger kaum weiter auffällt.

In Fig.3B ist eine weitere Ausführungsform des erfindungsgemäßen Innenohrstörungs-Behandlungsgeräts gezeigt, das sich von der ersten Ausführungsform dadurch unterscheidet, daß es sich bei der Befestigungsvorrichtung 320 um einen Bügel handelt, der beispielsweise ein speziell für das Innenohrstörungs-Behandlungsgerät gefertigtes Teil darstellt. Der Bügel 320 kann aber auch Teil eines Hörgeräts, eines Tinnitusmaskers oder eines Kombinationsgeräts aus einem Hörgerät und einem Tinnitusmasker sein. Im übrigen entspricht diese zweite Ausführungsform des Innenohrstörungs-Behandlungsgeräts der vorstehend beschriebenen ersten Ausführungsform, so daß bezüglich seiner technischen Einzelheiten auf die vorstehende Beschreibung verwiesen werden darf.

Gemäß einer weiteren, in den Figuren nicht näher gezeigten Ausführungsform der Erfindung kann die Low-Level-Laserbestrahlungsvorrichtung 310 eine separate Einheit sein, die beispielsweise in einer Tasche oder am Gürtel des Patienten getragen wird. In diesem Fall ist die Lichtleitervorrichtung 311 eine flexible Leitung, die in einer Linse endet, welche mittels der Befestigungsvorrichtung am Ohr befestigt werden kann. Diese Ausführungsform der Erfindung ist insbesondere dann von Vorteil, wenn die Laserbestrahlungsvorrichtung 310 und/oder deren Batterie vergleichsweise schwer ist.

Für die Low-Level-Laserbestrahlungsvorrichtung 310 kann ein (in den Figuren nicht gezeigter) Ein/Aus-Schalter vorgesehen werden, der es dem Patienten ermöglicht, die Behandlung zu beliebigen Zeiten vorzunehmen. Die Laserbestrahlungsvorrichtung 310 kann weiterhin eine manuelle Einstellvorrichtung aufweisen, mittels der ihre Ausgangsleistung und/oder die Wellenlänge des Laserstrahls auf einen geeigneten Wert eingestellt werden kann. Die Laserbestrahlungsvorrichtung 10 arbeitet entweder im kontinuierlichen oder im pulsierenden Betrieb, wobei gegebenenfalls eine Steuervorrichtung vorgesehen sein kann, mittels der die gewünschte Betriebsart und/oder die Impulsfrequenz eingestellt werden kann. Die Ausgangsleistung der Laserbestrahlungsvorrichtung 310 beträgt vorzugsweise zwischen 1 und 120 mW. Die Wellenlänge des Laserstrahls liegt im Bereich von 180 nm bis 1000 nm. Somit ist es möglich, handelsübliche Laserdioden als Strahlungsquelle zu verwenden.

Untersuchungen haben gezeigt, daß mit dem erfindungsgemäßen Gerät insbesondere dann gute Heilungserfolge erzielbar sind, wenn der Laserstrahl auf das Mastoid oder über den Gehörgang auf das Mittelohr einwirkt. Diese Bereiche des Ohres können durch geeignete Einstellung der Lichtleitervorrichtung 311 bestrahlt werden. Um eine noch bessere Heilungswirkung zu erzielen, kann das erfindungsgemäße Gerät gegebenenfalls dahingehend modifiziert werden, daß zwei Lichtleitervorrichtungen mit einer jeweiligen Linse vorgesehen werden, wobei die eine Linse auf das Mastoid und die andere Linse auf das Mittelohr einwirkt. Die Anzahl der Lichtleitervorrichtungen und Linsen kann selbstverständlich weiter erhöht werden, falls je nach der Art der Erkrankung des Innenohrs noch andere Bereiche des Ohres therapeutisch beaufschlagt werden sollen.

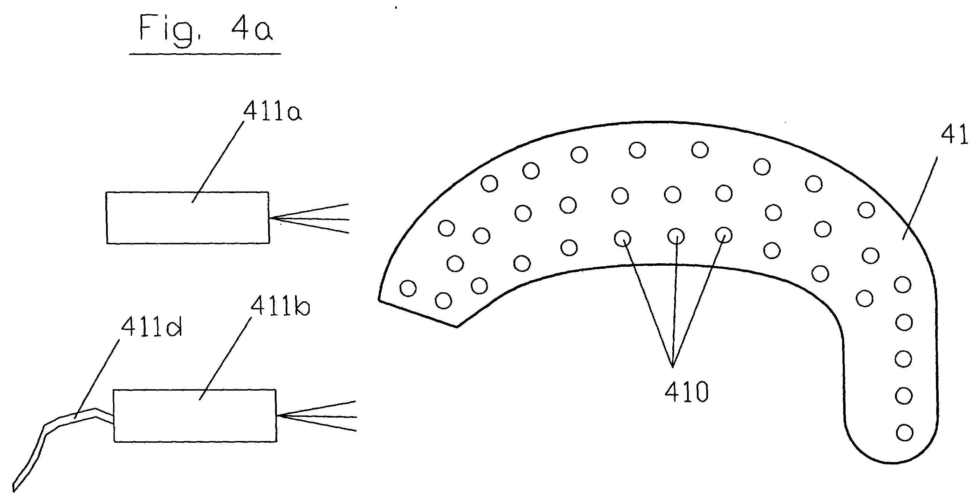

Gemäß Fig.4A besteht eine erste Ausführungsform des erfindungsgemäßen Geräts zur Stimulierung des Zentralnervensystems im wesentlichen aus einem Helm 41, der beispielsweise wie ein Fahrradhelm aus geschäumtem Material oder aus einem anderen geeigneten Kunststoffmaterial besteht. Der Helm 41 weist eine Vielzahl geeigneter Bohrungen auf, in denen jeweils eine Low-Level-Laserbestrahlungsvorrichtung 410 eingesetzt ist. Bei dieser Low-Level-Laserbestrahlungsvorrichtung 410 kann es sich entweder um eine Laserdiode 411a handeln, die eine eigene Stromversorgung wie beispielsweise eine Batterie besitzt, oder aber um eine Laserdiode 11b, die über eine Stromversorgungsleitung 411d mit einer zentralen (nicht gezeigten) Stromversorgungseinrichtung verbunden ist.

Im Inneren des Helms 41 sind mehrere (nicht gezeigte) Abstandshalter vorgesehen, die dafür Sorge tragen, daß die nach innen gerichteten Austrittsöffnungen der Laserdioden 411a bzw. 411b einen vorbestimmten Abstand zur Kopfhaut des Patienten aufweisen. Hierdurch wird erreicht, daß der der jeweiligen Laserdiode 411a bzw. 411b zugeordnete Bereich der Kopfhaut des Patienten gleichmäßig und mit definierter Energie bestrahlt wird.

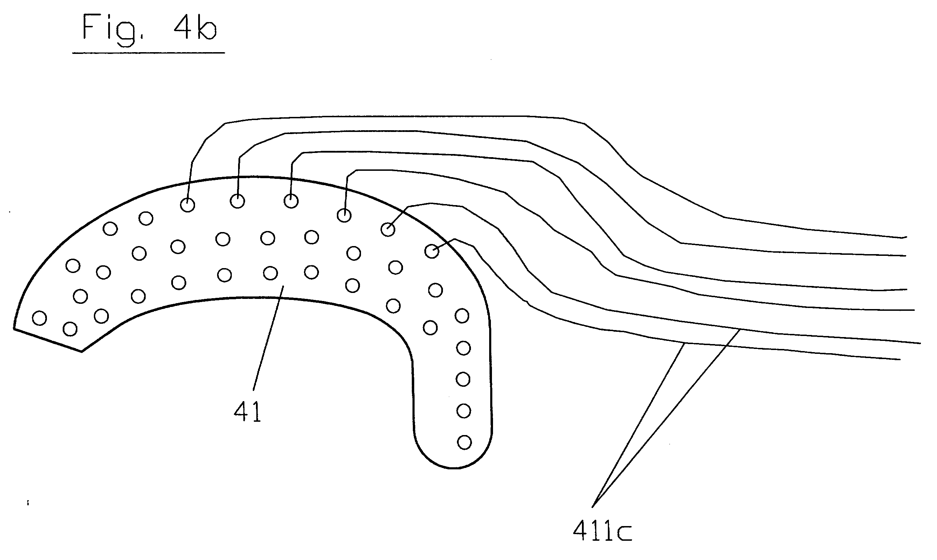

In Fig.4B ist eine weitere Ausführungsform des Helms gezeigt, die sich von der Ausführungsform der Fig.4B dadurch unterscheidet, daß als Laserstrahl-Sendeelemente Lichtleitervorrichtungen 411c vorgesehen sind, die beispielsweise von einer einzigen (nicht gezeigten) Lichtquelle gespeist werden. Alternativ hierzu kann für jedes oder zumindest für einen Teil der Lichtleitervorrichtungen 411c eine eigene Laserlichtquelle vorgesehen werden, so daß die Möglichkeit besteht, einige oder alle Lichtleitervorrichtungen 411c mit anderer Frequenz und/oder Leistung zu betreiben. Die Laserlichtquellen können entweder Teil des Helms 41 sein oder in einem externen Gerät angeordnet werden.

Die in Fig.4B gezeigte Variante des Helms hat somit den Vorteil, daß die Wellenlänge und/oder Energie der Laserstrahlen zentral gesteuert werden kann, wodurch die Ansteuerelektronik gegebenenfalls vereinfacht werden kann. Am im Inneren des Helms 41 liegenden Ende jeder Lichtleitervorrichtung 411c sitzt vorzugsweise eine (nicht gezeigte) Linse, die eine noch günstigere bzw. gleichmäßigere Verteilung des abgegebenen Laserlichts ermöglicht.

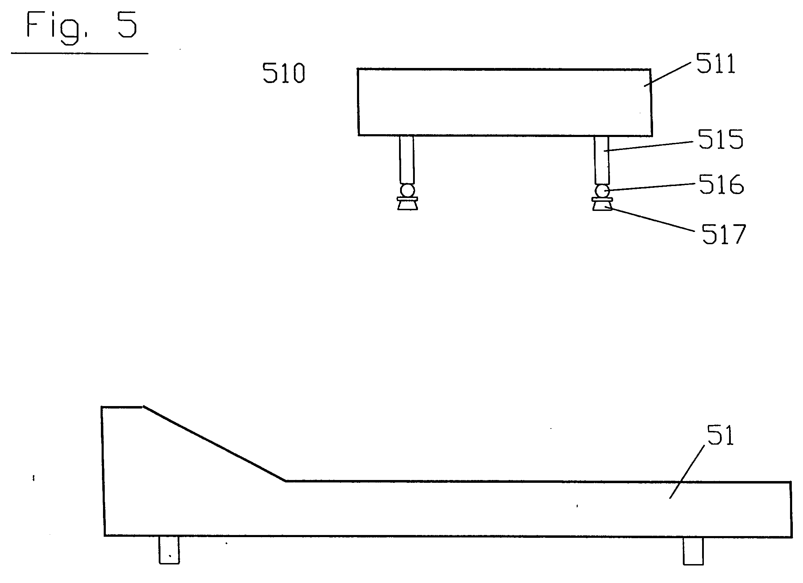

Wie aus der Fig.5 hervorgeht, besteht das erfindungsgemäße Gerät im wesentlichen aus einem Gehäuse 11, das erfindungsgemäße Gerät zur Therapie und Prophylaxe von Dekubitus beispielsweise aus Metall oder einem geeigneten Kunststoffmaterial besteht. Das Gehäuse 511 weist eine Vielzahl geeigneter Bohrungen auf, in denen jeweils eine Rohr 515 eingesetzt ist, das zusammen mit einem Gelenk 516 einen schwenkbaren Halter bildet. An diesem schwenkbaren Halter ist jeweils ein Laserstrahl-Sendeelement 517 befestigt, das beispielsweise aus einer Laserdiode besteht. Sämtliche Laserstrahl-Sendeelemente bzw. Dioden 517 bilden die Low-Level-Laserbestrahlungsvorrichtung 510. Die Laserstrahl-Sendeelemente 517 weisen ferner eine lediglich schematisch angedeutete Streulinse zur Fächerung des von ihnen abgegebenen Laserstrahls auf.

Das Gehäüse 511 ist mittels nicht näher gezeigter Einrichtungen an der Decke oder an einem geeigneten Ständer befestigt; das Gehäuse 511 beherbergt ferner sämtliche elektrischen und elektronischen Komponenten zur Ansteuerung der Laserstrahl-Sendeelemente 517. Zum Einschalten des Geräts ist ein (nicht gezeigter) Ein/Aus-Schalter vorgesehen, während die Ausgangsleistung der Laserstrahl-Sendeelemente 517 mittels eines Drehreglers eingestellt werden kann.

Wie aus der Fig.5 ersichtlich ist, werden die Laserstrahl-Sendeelemente 517 mittels der schwenkbaren Halter 515, 516 auf einen (nicht gezeigten) Patienten gerichtet, der sich auf einer Liege bzw. einem Bett 51 befindet. Durch die Vielzahl der Laserstrahl-Sendeelemente 517 sowie aufgrund der mit den Streulinsen erzeugten Fächerung der Laserstrahlen kann eine gleichmäßige und ausreichende therapeutische Behandlung der erkrankten bzw. wundgelegenen Haut des Patienten mit wenigen Handgriffen erreicht werden.

Anstelle separater Laserstrahl-Sendeelemente in Form von Laserdioden 517 können auch (nicht gezeigte) Lichtleitervorrichtungen vorgesehen werden, die beispielsweise von einer einzigen (nicht gezeigten) Lichtquelle gespeist werden. Die Verwendung von Lichtleitern hat darüber hinaus den Vorteil, daß die Wellenlänge und/oder Energie der Laserstrahlen zentral gesteuert werden kann, wodurch die Ansteuerelektronik gegebenenfalls vereinfacht werden kann. Die Laserstrahl-Sendeelemente 517 erzeugen einen Laserstrahl mit einer Wellenlänge im Bereich von 180 nm bis 1000 nm, wobei die Ausgangsleistung vorzugsweise zwischen 1 mW und 120 mW liegt.

Das Gehäuse 511 beinhaltet ferner eine (nicht gezeigte) manuell bedienbare Zeitschaltuhr, die nach Ablauf einer vorwählbaren Zeitspanne die Laserstrahl-Sendeelemente 517 deaktiviert. Der Therapeut kann somit nach geeigneter Einstellung bzw. Justierung des Geräts den Patienten bis zum Ablauf der vorgewählten Zeitspanne alleine lassen. Als günstig haben sich in der Praxis Behandlungszeiträume von bis zu 30 Minuten erwiesen.

Die Laserstrahl-Sendeelemente 517 bzw. ihre Gelenke 516 können einen (nicht gezeigten) motorischen Antrieb aufweisen, der den Abstrahlwinkel der Laserstrahl-Sendeelemente 517 ändert, so daß der abgegebene Laserstrahl einen entsprechenden Bereich der Hautoberfläche gleichsam abtastet. Hierdurch ist es möglich, diesen Bereich der Haut des Patienten in zyklischer Folge mit den Laserstrahlen zu beaufschlagen. Somit kann die therapeutische Wirkung ggf. vergleichmäßigt werden. Ferner ist es hierdurch möglich, Laserstrahl-Sendeelemente 517 mit größerer Leistung einzusetzen, da die jeweiligen Hautbereiche aufgrund der motorischen Verstellung nur kurzzeitig beaufschlagt werden.

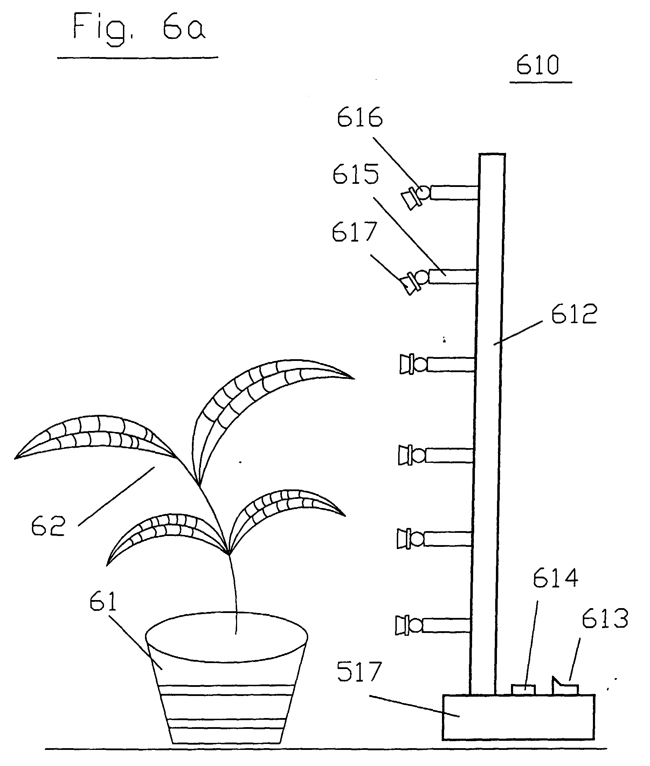

Gemäß Fig.6A besteht eine Ausführungsform eines Geräts zur Biostimulation von Pflanzen im wesentlichen aus einem Ständer 612, der beispielsweise aus einem Rohr aus Metall oder einem geeigneten Kunststoffmaterial besteht. Der Ständer 612 weist eine Vielzahl geeigneter Bohrungen auf, in denen jeweils eine waagrecht verlaufende Stange 615 eingesetzt ist, die zusammen mit einem Gelenk 616 einen schwenkbaren Halter bildet. An diesem schwenkbaren Halter ist jeweils ein Laserstrahl-Sendeelement 617 befestigt, das beispielsweise aus einer Laserdiode besteht. Sämtliche Laserstrahl-Sendeelemente bzw. Dioden 617 bilden die erfindungsgemäße Low-Level-Laserbestrahlungsvorrichtung 10. Die Laserstrahl-Sendeelemente 617 weisen ferner eine lediglich schematisch angedeutete Streulinse zur Fächerung des von ihnen abgegebenen Laserstrahls auf.

Der Ständer 612 ist in einem Gehäüse 611 befestigt, das gleichzeitig als Standfuß dient und sämtliche elektrischen und elektronischen Komponenten zur Ansteuerung der Laserstrahl-Sendeelemente 617 beherbergt. Zum Einschalten des Geräts ist ein Ein/Aus-Schalter 613 vorgesehen, während die Ausgangsleistung der Laserstrahl-Sendeelemente 617 mittels eines Drehreglers 614 eingestellt werden kann.

Wie aus der Fig.6A ersichtlich ist, werden die Laserstrahl-Sendeelemente 617 mittels der schwenkbaren Halter 615, 616 auf eine Pflanze 62 gerichtet, die sich in einem Topf 61 befindet. Durch die Vielzahl der Laserstrahl-Sendeelemente 617 sowie aufgrund der mit den Streulinsen erzeugten Fächerung der Laserstrahlen kann eine gleichmäßige und ausreichende Biostimulierung der Pflanze 2 mit wenigen Handgriffen erreicht werden.

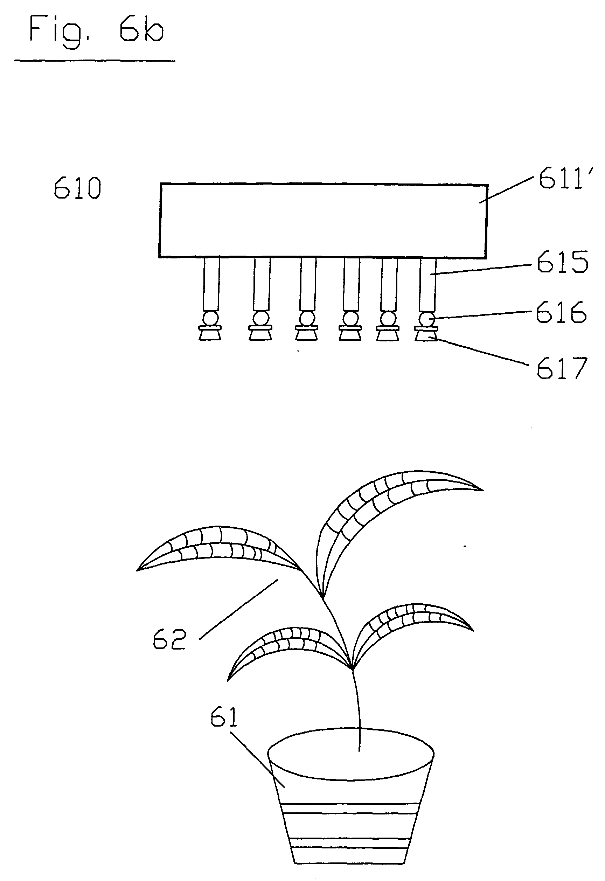

In Fig.6B ist eine weitere Ausführungsform des erfindungsgemäßen Geräts gezeigt, das sich von der Ausführungsform der Fig.6A dadurch unterscheidet, daß die Laserstrahl-Sendeelemente 617 samt ihren schwankbaren Haltern 615, 616 an einem Gehäuse 611' befestigt sind, das zur Deckenmontage vorgesehen ist. Im übrigen arbeitet dieses Gerät in gleicher Weise wie das Gerät der Fig.6A.

Anstelle separater Laserstrahl-Sendeelemente in Form von Laserdioden 617 können auch (nicht gezeigte) Lichtleitervorrichtungen vorgesehen werden, die beispielsweise von einer einzigen (nicht gezeigten) Lichtquelle gespeist werden. Diese Ausführungsform bietet sich ggf. für größere Räume wie Gewächshäuser und dergleichen an. Die Verwendung von Lichtleitern hat darüber hinaus den Vorteil, daß die Wellenlänge und/oder Energie der Laserstrahlen zentral gesteuert werden kann, wodurch die Ansteuerelektronik gegebenenfalls vereinfacht werden kann.

Die Laserstrahl-Sendeelemente 617 erzeugen einen Laserstrahl mit einer Wellenlänge im Bereich von 180 nm bis 1000 nm, wobei die Ausgangsleistung vorzugsweise zwischen 1 mW und 500 mW liegt.

Das Gehäuse 611 bzw. 611' beinhaltet ferner eine (nicht gezeigte) manuell bedienbare oder automatisch arbeitende Steuereinrichtung, die in Abhängigkeit von der Wachstumsphase der Pflanze 61 eines oder mehrere der Laserstrahl-Sendeelemente 617 mit jeweils geeigneter Wellenlänge aktiviert und/oder deren Ausgangsleistung ändert. Somit ist eine optimale Anpassung an das Wachstum der Pflanze 61 möglich. Die Einstellung der Wellenlänge bzw. der Ausgangsleistung kann ggf. auch über eine Zeitsteuerung erfolgen, wenn das Wachstumsverhalten der Pflanze 61 bekannt ist.

## Patentansprüche

1. Gerät (10) zur Therapie von Rhinitis,
gekennzeichnet durch
eine in einem Einhandgehäuse (11) befindliche Low-Level-Laserbestrahlungsvorrichtung (14, 16), die mindestens einen Laserstrahl erzeugt, der über eine im wesentlichen längliche, in die Nasenöffnung eines Patienten einführbare Lichtleitervorrichtung (20) auf das Naseninnere einwirkt.

2. Gerät nach Anspruch 1, dadurch gekennzeichnet, daß die Low-Level-Laserbestrahlungsvorrichtung (14, 16) einen Laserstrahl mit einer Wellenlänge im Bereich von 180 nm bis 1000 nm erzeugt.

3. Gerät nach Anspruch 1 oder 2, dadurch gekennzeichnet, daß die Low-Level-Laserbestrahlungsvorrichtung (14, 16) einen Laserstrahl mit einer Ausgangsleistung zwischen 1 mW und 5 mW (Klasse 3A) abgibt.

4. Gerät nach einem der Ansprüche 1 bis 3, dadurch gekennzeichnet, daß die Low-Level-Laserbestrahlungsvorrichtung mindestens ein Laserstrahl-Sendeelement in Form einer Laserdiode (16) aufweist.

5. Gerät nach einem der Ansprüche 1 bis 4, dadurch gekennzeichnet, daß am austrittseitigen Ende der Lichtleitervorrichtung (20) eine Streulinse (21) zur Fächerung des Laserstrahls sitzt.

6. Gerät nach einem der Ansprüche 1 bis 5, dadurch gekennzeichnet, daß zur Stromversorgung der Low-Level-Laserbestrahlungsvorrichtung (14, 16) eine im Einhandgehäuse (11) befindliche Batterie (5) vorgesehen ist.

7. Gerät nach Anspruch 6, dadurch gekennzeichnet, daß die Batterie (5) aufladbar ist.
